# EUROPEAN PATENT APPLICATION

(11) **EP 4 115 974 A1**
(43) Date of publication of application: **11.01.2023**
(21) Application number: 21764548.0
(22) Date of filing: 01.03.2021
(51) Int. Cl.: B01J 23/75, B01J 23/83, B01J 23/847, B01J 37/02, B01J 37/08, B01J 37/18, C07B 61/00, C07C 209/26, C07C 211/27, C07C 211/29, C07C 211/35, C07C 213/02, C07C 217/58, C07D 207/335, C07D 307/52

(54) **OXIDE-SUPPORTED COBALT CATALYST CAPABLE OF ACCELERATING REDUCTIVE AMINATION**

(30) Priority: 04.03.2020 JP 2020036868
(71) Applicant: Tokyo Institute of Technology, Tokyo 152-8550 (JP)
(72) Inventor: HARA, Michikazu, Tokyo 152-8550 (JP); KAMATA, Keigo, Tokyo 152-8550 (JP); KITA, Yusuke, Tokyo 152-8550 (JP); DENG Dian, Tokyo 152-8550 (JP)
(74) Representative: J A Kemp LLP
(86) International application number: PCT/JP2021/007662
(87) International publication number: WO 2021/177219

(57) **Abstract**

In order to enable a reductive amination reaction at a low temperature and a low hydrogen pressure, provided is a catalyst comprising cobalt supported on an oxide, the catalyst produced by a method comprising the following steps (1) to (4): (1) a step of mixing a salt containing a cobalt ion and an oxide in water, (2) a step of distilling water away from a mixed solution obtained in step (1) and drying a resulting solid material, (3) a step of calcining a dried material obtained in step (2) in a nitrogen stream, and (4) a step of reducing a calcined product obtained in step (3) in a hydrogen stream.

## Description

### [Technical Field]

The present invention relates to a novel catalyst. The catalyst of the present invention can efficiently accelerate a reductive amination reaction at a low temperature and a low hydrogen pressure.

### [Background Art]

Amines widely used as synthetic intermediates in various fields can be synthesized by a reductive amination reaction, in which a carbonyl compound is allowed to react with a nitrogen source in the presence of a reducing agent to thereby cause alkylation of the nitrogen source. In particular, reductive amination involving use of a hydrogen molecule as a reducing agent has been utilized for industrial amine synthesis in view of excellent environmental friendliness because of a coproduct of water only and ease of control of the reaction.

As a catalyst for the reductive amination reaction involving use of hydrogen as a reducing agent, a precious metal catalyst, typically ruthenium or rhodium, has been mainly used so far. In view of elements strategy, studies on non-precious metals as catalysts have been promoted, but the catalysts as previously reported need high temperature/high pressure. In a reductive amination reaction of furfural and ammonia, Rh/Al₂O₃ and Ru/Nb₂O₅, which are existing heterogeneous precious metal catalysts, need a temperature of 80 to 90°C and a hydrogen pressure of 2 to 4 MPa (Non Patent Literatures 1 and 2).

Recently, cobalt, which is a non-precious metal, has received attention as a catalyst for a reductive amination reaction. For example, Patent Literature 1 discloses a method for synthesizing isophorone diamine from isophorone nitrile with a catalyst including metallic cobalt as an active constituent and activated carbon as a carrier. Moreover, Patent Literature 1 also discloses, as a comparative example for this catalyst including activated carbon as a carrier, a catalyst including an oxide, such as CaO, Al₂O₃, SiO₂ or TiO₂, as a carrier instead of activated carbon (paragraph [0056]). The catalyst including activated carbon as a carrier is prepared by carrying out calcining in a nitrogen atmosphere (paragraph [0040]), and on the other hand, the catalyst including an oxide as a carrier is prepared by carrying out calcining in an air atmosphere (paragraph [0052]). In the case of using any of the catalysts, the synthesis reaction is carried out at a relatively high temperature of 80°C and a high hydrogen partial pressure of 8 MPa (paragraphs [0042] and [0047]).

### [Citation List]

### [Patent Literature]

[Patent Literature 1] Chinese Patent Application Publication No. 108686660

### [Non Patent Literature]

[Non Patent Literature 1] Kawanami et al. Green Chem. 2016, 18, 487.
[Non Patent Literature 2] Hara et al. J. Am. Chem. Soc. 2017, 139, 11493.

### [Summary of Invention]

### [Technical Problem]

The conventional reductive amination reaction needs to be carried out at a high temperature and a high hydrogen pressure, as described above. However, for the reaction at a high temperature and a high hydrogen pressure, facilities having heat resistance and pressure resistance are necessary, and this is undesirable from the economical and safety viewpoints. The present invention has been made in such circumstance as above, and it is an object of the present invention to provide means that enables a reductive amination reaction at a low temperature and a low hydrogen pressure (50°C, about 0.5 MPa).

### [Solution to Problem]

As a result of earnest studies for solving the above problem, the present inventor has has found that a catalyst including cobalt supported on an oxide efficiently accelerates a reductive amination reaction at a low temperature and a low hydrogen pressure, and has completed the present invention.

Specifically, the present invention provides [1] to [15].
[1] A catalyst comprising cobalt supported on an oxide, the catalyst produced by a method comprising the following steps (1) to (4):
   (1) a step of mixing a salt containing a cobalt ion and an oxide in water,
   (2) a step of distilling water away from a mixed solution obtained in step (1) and drying a resulting solid material,
   (3) a step of calcining a dried material obtained in step (2) in a nitrogen stream, and
   (4) a step of reducing a calcined product obtained in step (3) in a hydrogen stream.
[2] The catalyst according to [1], wherein an abundance ratio of metallic cobalt in a surface of the cobalt supported on the oxide is 50% or more.
[3] The catalyst according to [1] or [2], wherein a ratio of the number of atoms of cobalt on a catalyst surface to the number of atoms of the cobalt and an oxidized element on the catalyst surface is 4% or less.
[4] The catalyst according to any one of [1] to [3], wherein the catalyst is used in a reductive amination reaction.
[5] The catalyst according to any one of [1] to [4], wherein the oxide is SiO₂, ZrO₂, Nb₂O₅, TiO₂, Al₂O₃, CeO₂, or MgO.
[6] The catalyst according to any one of [1] to [4], wherein the oxide is SiO₂.
[7] The catalyst according to any one of [1] to [4], wherein the oxide used in step (1) is SiO₂, and an amount of silanol groups in a surface of the SiO₂ is 4 mmol/g or less.
[8] The catalyst according to any one of [1] to [4], wherein the oxide used in step (1) is SiO₂, and a surface area of the SiO₂ is 100 m²g⁻¹ or more.
[9] The catalyst according to any one of [1] to [4], wherein the oxide used in step (1) is SiO₂, an amount of silanol groups in a surface of the SiO₂ is 4 mmol/g or less, and a surface area of the SiO₂ is 100 m²g⁻¹ or more.
[10] The catalyst according to any one of [1] to [4], wherein the oxide used in step (1) is SiO₂, and the SiO₂ is SiO₂ having been subjected to heat treatment.
[11] The catalyst according to [10], wherein the heat treatment is heat treatment at 350 to 480°C for 3 to 8 hours.
[12] A method for producing an amine, comprising a step of obtaining, from a carbonyl compound represented by the general formula (I): 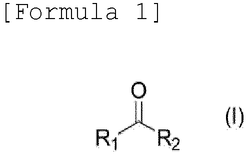 wherein R₁ and R₂ are the same or different and each represent a hydrogen atom, an aryl group optionally substituted by a substituent, or an alkyl group optionally substituted by a substituent, and R₁ and R₂ are optionally bonded to each other to form a ring,
   an amine represented by the general formula (II): 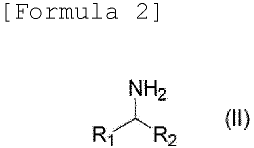 wherein R₁ and R₂ have the same meanings as above,
   in the presence of the catalyst according to any one of [1] to [11] and a reducing agent.
[13] The method for producing an amine according to [12], wherein the reducing agent is a hydrogen molecule.
[14] The method for producing an amine according to [13], wherein the step of obtaining, from a carbonyl compound represented by the general formula (I), an amine represented by the general formula (II) is carried out at a temperature of 100°C or less and a hydrogen pressure of 1.0 MPa or less.
[15] The method for producing an amine according to [14], wherein the carbonyl compound represented by the general formula (I) is furfural, and the amine represented by the general formula (II) is furfurylamine.

The present application claims priority to Japanese patent application JP Application No. 2020-036868, the contents disclosed in the specification and/or the drawings of which are incorporated herein.

### [Advantageous Effect of Invention]

The present invention provides a novel catalyst. The catalyst of the present invention can efficiently accelerate a reductive amination reaction at a low temperature and a low hydrogen pressure.

### [Brief Description of Drawings]

[Figure 1] Figure 1 shows results of a study on reusability of a Co/SiO₂ catalyst.
[Figure 2] Figure 2 shows electron microphotographs of a Co/SiO₂ catalyst before the reaction (left) and after reuses of 4 times (right).
[Figure 3] Figure 3 shows XRD patterns of a Co/SiO₂ catalyst. The reduction temperature was changed.
[Figure 4] Figure 4 shows XPS spectra of Co/SiO₂ and CoO/SiO₂.

### [Description of Embodiments]

Hereinafter, the present invention will be described in detail.

### (A) Catalyst

The catalyst of the present invention is a catalyst comprising cobalt supported on an oxide, and is characterized in that the catalyst is produced by a method comprising the following steps (1) to (4).

In step (1), a salt containing a cobalt ion and an oxide are mixed in water.

The salt containing a cobalt ion is not particularly limited, and for example, cobalt nitrate, cobalt chloride, cobalt sulfate, or the like can be used.

The oxide is not particularly limited either, and it may be a metallic oxide or a non-metallic oxide. Specific examples include SiO₂, ZrO₂, Nb₂O₅, TiO₂, Al₂O₃, CeO₂, and MgO. The oxide is preferably one that hardly forms a composite oxide together with cobalt, and from such a viewpoint, SiO₂ is preferable.

The SiO₂ used as a raw material herein is preferably one of which the amount of silanol groups in the surface is small. The reason is that the silanol group in the surface of SiO₂ reacts with cobalt to form cobalt oxide, and therefore, if the amount of the silanol groups in the surface is large, the ratio of metallic cobalt (Co⁰) in the cobalt to be supported on SiO₂ is decreased, which results in reduced catalytic activity. Specifically, the amount of silanol groups in the surface is preferably 4 mmol/g or less, more preferably 3.5 mmol/g or less, and still more preferably 3 mmol/g or less.

The SiO₂ used as a raw material is preferably one having a large surface area. The reason is that if the surface area is small, the catalytic activity is reduced. Specifically, the surface area is preferably 100 m²g⁻¹ or more, more preferably 110 m²g⁻¹ or more, and still more preferably 120 m²g⁻¹ or more.

The SiO₂ having a small amount of silanol groups in the surface and having a large surface area is obtained by subjecting SiO₂ to heat treatment. The temperature in this heat treatment is preferably about 400°C. More specifically, the temperature is preferably 300 to 480°C, and more preferably 350 to 450°C. The heating time in the heat treatment is preferably about 5 hours. More specifically, the heating time is preferably 3 to 8 hours, and more preferably 4 to 6 hours.

The mixing ratio between the salt containing a cobalt ion and the oxide is not particularly limited. The mixing ratio is preferably such that the ratio of cobalt to the oxide to about 20 wt%, and for example, the ratio can be 5 to 50 wt%, 10 to 30 wt%, or 15 to 25 wt%.

In step (2), water is distilled away from the mixed solution obtained in step (1), and the resulting solid material is dried.

The distillation of water can be carried out in accordance with a conventional method, and for example, it can be carried out using a rotary evaporator. The drying can also be carried out in accordance with a conventional method, and for example, it can be carried out by heating under reduced pressure.

In step (3), the dried material obtained in step (2) is calcined in a nitrogen stream.

The temperature in the calcining is not particularly limited, and for example, it can be 200 to 600°C, 300 to 500°C, or 350 to 450°C. The calcining time is not particularly limited either, and for example, it can be 0.5 to 4 hours, 1 to 3 hours, or 1.5 to 2.5 hours.

In step (4), the calcined product obtained in step (3) is reduced in a hydrogen stream.

The reduction in a hydrogen stream is carried out under high temperature conditions. The temperature of the high temperature conditions may be a temperature at which cobalt oxide can be reduced to metallic cobalt, and for example, it can be 400 to 800°C, 500 to 700°C, or 550 to 650°C. The time for performing reduction is not particularly limited either, and for example, it can be 0.5 to 4 hours, 1 to 3 hours, or 1.5 to 2.5 hours.

Herein, the "catalyst," which is a "product," is not specified by its structure or characteristics but is specified by its production method as described above. The reason for this is that the structure or the characteristics of the cobalt supported on the oxide are extremely complicated, and the work to identify them needs significantly excessive economic spending and time.

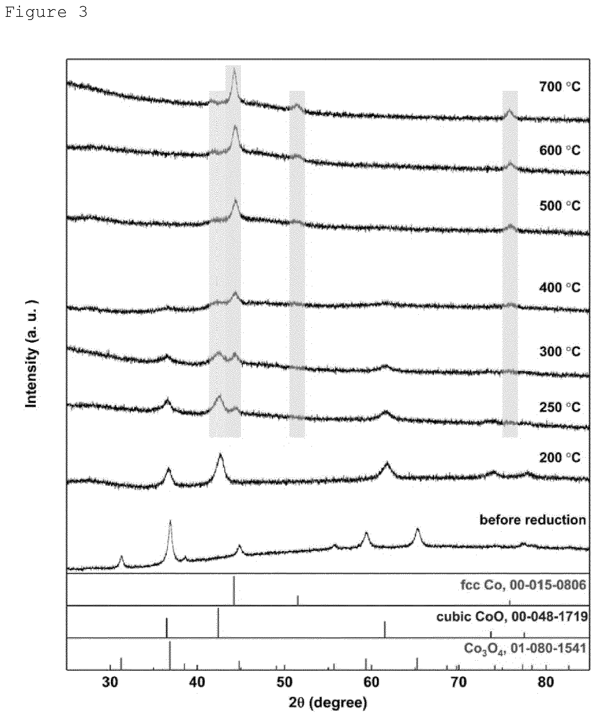

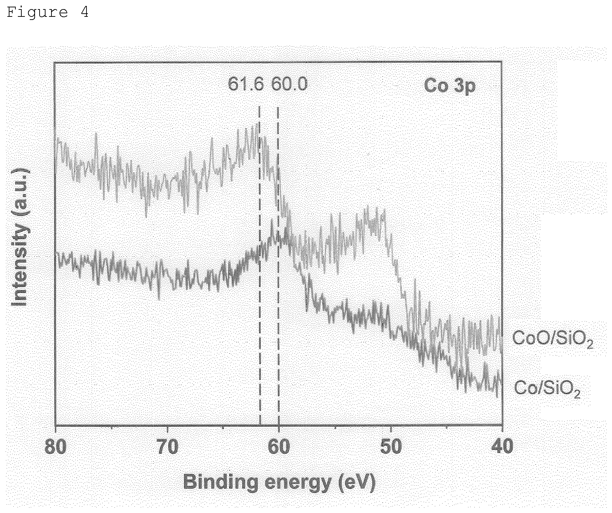

In the catalyst of the present invention, the cobalt supported on the oxide contains a large amount of metallic cobalt (Co⁰). The catalyst of the present invention exhibits a high activity even at a low temperature and a low hydrogen pressure, and it is thought that containing a large amount of metallic cobalt is one of the reasons. The "cobalt containing a large amount of metallic cobalt" means, for example, cobalt in which an abundance ratio of metallic cobalt in the surface of the cobalt supported on the oxide is 50% or more, and preferably 60% or more. Here, the abundance ratio of the metallic cobalt is estimated from an X-ray photoelectron spectroscopy (XPS) spectrum. In the cobalt in the catalyst, not only metallic cobalt (Co⁰) but also cobalt oxide (Co²⁺) is contained. Regarding the metallic cobalt and the cobalt oxide, different peaks are detected in XPS spectra and X-ray diffraction (XRD) patterns, and therefore, the "cobalt containing a large amount of metallic cobalt" can also be defined by these peaks. For example, in XPS spectra, a peak of the metallic cobalt is detected at or in the vicinity of 60 eV (or 59 eV), and a peak of the cobalt(II) oxide is detected at or in the vicinity of 62 eV, as shown in Figure 4. Accordingly, cobalt whose peak is detected at or in the vicinity of 60 eV (or 59 eV) and not detected at or in the vicinity of 62 eV can be defined as "cobalt containing a large amount of metallic cobalt". As shown in Figure 3, for 00-015-0806, which is a standard XRD pattern of metallic cobalt, peaks are detected in the vicinity of 45°, in the vicinity of 53°, and in the vicinity of 76°, and for 00-048-1719, which is a standard XRD pattern of cobalt(II) oxide, peaks are detected in the vicinity of 36°, in the vicinity of 43°, and in the vicinity of 62°. Accordingly, cobalt whose peaks are detected at or in the vicinity of 45°, 53°, and 76° in an XRD pattern, or cobalt whose peaks are detected at or in the vicinity of 45°, 53°, and 76°, and not detected at or in the vicinity of 36°, 43°, or 62° in an XRD pattern can also be defined as "cobalt containing a large amount of metallic cobalt".

It is thought that a small number of atoms of cobalt on a catalyst surface is also one of the reasons why the catalyst of the present invention exhibits a high activity at a low temperature and a low hydrogen pressure. Here, "a small number of atoms of cobalt on a catalyst surface" means that a ratio of the number of atoms of cobalt on the catalyst surface to the number of atoms of the cobalt and an oxidized element on the catalyst surface is, for example, 4% or less, 3% or less, or 2% or less. The "oxidized element" refers to an element been oxidized in an oxide (e.g., silicon in silicon dioxide).

The catalyst of the present invention is mainly used for a reductive amination reaction, but it may be used for other reactions.

### (B) Method for producing amine

The method for producing an amine of the present invention is a method for producing an amine by a reductive amination reaction using the above-described catalyst of the present invention. More specifically, the method of the present invention is a method for producing an amine, comprising a step of obtaining, from a carbonyl compound represented by the general formula (I) : 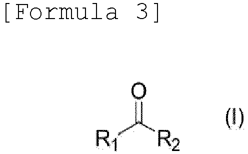 wherein R₁ and R₂ are the same or different and each represent a hydrogen atom, an aryl group optionally substituted by a substituent, or an alkyl group optionally substituted by a substituent, and R₁ and R₂ are optionally bonded to each other to form a ring,
an amine represented by the general formula (II): 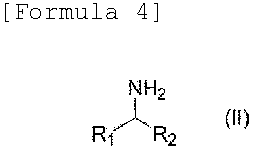 wherein R₁ and R₂ have the same meanings as above,
in the presence of the catalyst of the present invention and a reducing agent.

Examples of the "aryl group" in the present invention include a phenyl group, a naphthalen-1-yl group, a naphthalen-2-yl group, a pyridinyl group (pyridin-2-yl group, pyridin-3-yl group, pyridin-4-yl group), a pyrimidinyl group (pyrimidin-2-yl group, pyrimidin-4-yl group, pyrimidin-5-yl group), a furanyl group (furan-2-yl group, furan-3-yl group), a thienyl group (thiophen-2-yl group, thiophen-3-yl group), and a pyrrolyl group (pyrrol-2-yl group, pyrrol-3-yl group).

Examples of the "alkyl group" in the present invention include a methyl group, an ethyl group, an n-propyl group, an isopropyl group, an n-butyl group, an isobutyl group, a sec-butyl group, a tert-butyl group, and an n-pentyl group.

Examples of the "substituent" in the "aryl group optionally substituted by a substituent" and the "alkyl group optionally substituted by a substituent" in the present invention include a methyl group, an ethyl group, a methoxy group, an ethoxy group, a fluorine atom, a chlorine atom, a bromine atom, an iodine atom, a trifluoromethyl group, and a hydroxymethyl group.

R₁ and R₂ in the general formula (I) and the general formula (II) are each a hydrogen atom, an aryl group optionally substituted by a substituent, or an alkyl group optionally substituted by a substituent. Preferably, one is a hydrogen atom, and the other is an aryl group optionally substituted by a substituent. More preferably, one is a hydrogen atom, and the other is a phenyl group optionally substituted by a substituent or a furanyl group optionally substituted by a substituent. Still more preferably, one is a hydrogen atom, and the other is a furanyl group.

R₁ and R₂ in the general formula (I) and the general formula (II) are optionally bonded to each other to form a ring such as cyclohexane.

A preferred carbonyl compound represented by the general formula (I) is, for example, furfural, and a preferred amine represented by the general formula (II) is, for example, furfurylamine.

The reducing agent may be one generally used in a reductive amination reaction, but it is preferable to use a hydrogen molecule.

The amount of the catalyst used is not particularly limited, and the catalyst can be used in an amount of, for example, 20 to 60 mg, or 30 to 50 mg, per 1 mmol of the carbonyl compound represented by the general formula (I).

The step of obtaining, from a carbonyl compound represented by the general formula (I), an amine represented by the general formula (II) is preferably carried out at a low temperature and a low hydrogen pressure. For example, the temperature can be 100°C or less, 90°C or less, 80°C or less, 70°C or less, or 60°C or less. The hydrogen pressure can be 1.0 MPa or less, 0.9 MPa or less, 0.8 MPa or less, 0.7 MPa or less, or 0.6 MPa or less.

### [Examples]

Hereinafter, the present invention will be described in more detail with reference to examples, but the present invention is in no way limited to these examples.

### [Example 1]

### (A) Material and method

### (1) Reagent

Cobalt nitrate hexahydrate was purchased from Kanto Chemical Co., Inc. SiO₂ (CARiACT Q-10) was provided by Fuji Silysia Chemical Ltd., MgO was provided by UBE Corporation, and TiO₂ (ST-01) was provided by ISHIHARA SANGYO KAISHA, LTD. As ZrO₂ (JRC-ZRO-6), Al₂O₃ (JRC-ALO-9) and CeO₂ (JRC-CEO-5), reference catalysts of the Catalysis Society of Japan were used. Nb₂O₅ was prepared by calcining hydrous niobic acid (provided by Companhia Brasileira de Metalurgia e Mineracao) in air at 400°C. The amount of silanol groups in the surface of SiO₂ (CARiACT Q-10) was measured, and as a result, it was 3 mmol/g.

### (2) Method for preparing 20 wt% supported cobalt catalyst

In 50 mL of distilled water, cobalt nitrate hexahydrate (1.23 g) and a carrier (1 g) were dispersed, and they were stirred at room temperature for 1 hour. Water was distilled away by a rotary evaporator, and then, the residue was dried by heating (100°C) under reduced pressure (to 0.25 kPa). The dry powder was calcined at 400°C for 2 hours in a nitrogen stream, thereby obtaining a precursor. The precursor was subjected to hydrogen reduction (30 mL/min) at 600°C for 2 hours, thereby preparing a supported cobalt catalyst. After the hydrogen reduction, the catalyst was transferred into a glove box without exposure to air, and it was used for a catalytic reaction.

### (3) Reductive amination reaction

In the glove box, 20 mg of the supported cobalt catalyst was weighed into a Teflon inner cylinder, and 0.5 mmol of furfural and 5 mL of toluene were added. The inner cylinder containing the reaction solution was set in a stainless-steel autoclave. The autoclave was taken out of the glove box, and the autoclave was purged with ammonia three times and then pressurized to 0.1 MPa. From above, hydrogen was introduced in such a manner that the total pressure became 0.5 MPa. The autoclave was heated and stirred at 50°C. After the reaction, the autoclave was allowed to cool down to room temperature and then depressurized. Thereafter, to the reaction solution, monochlorobenzene was added as internal standard, and a conversion and a yield were determined by gas chromatography.

### (4) Reuse experiment

The reductive amination reaction was carried out by the above-described method. After the reaction, the autoclave was placed in a glove box and depressurized. The catalyst was recovered by magnet and washed with toluene. To the reaction solution, monochlorobenzene was added as internal standard, and a conversion and a yield were determined by gas chromatography. Using the recovered catalyst, a reductive amination reaction was carried out in the same manner as above.

### (B) Results

### (1) Effect of carrier

Supported cobalt catalysts having different carriers were prepared, and a reductive amination reaction of furfural was carried out to examine influence of the carriers on the catalytic activity (Table 1). In the case of a catalyst having SiO₂ as a carrier, the targeted furfurylamine (1) was obtained in a yield of 74% (entry 1). On the other hand, in the case of other carriers, the targeted product was not obtained, and furfurylidene furfurylamine (2), which was an intermediate, or a trimer (3), which was a by-product, was only obtained (entries 2 to 7).

**[Table 1]**

| **Effect of carrier** | | | | | |
|---|---|---|---|---|---|
| 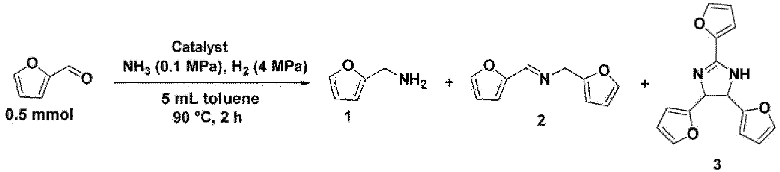 | | | | | |
| Entry | Catalyst | Conv. (%) | Yield (%) | | |
| | | | **1** | **2** | **3** |
| 1 | 5 wt% Co/SiO₂ | >99 | 74 | 19 | 1 |
| 2 | 5 wt% Co/ZrO₂ | 76 | - | 54 | 8 |
| 3 | 5 wt% Co/Nb₂O₅ | 85 | - | 39 | 9 |
| 4 | 5 wt% Co/TiO₂ | 59 | - | 16 | 17 |
| 5 | 5 wt% Co/Al₂O₃ | 62 | - | 1 | 30 |
| 6 | 5 wt% Co/CeO₂ | 39 | - | 34 | 5 |
| 7 | 5 wt% Co/MgO | 51 | - | 1 | 18 |

### (2) Influence of amount of cobalt supported

SiO₂ was fixed as a carrier, and catalysts having different amounts of cobalt supported were prepared. The catalytic amounts were adjusted in such a manner that the amounts of cobalt were the same as one another, and a reductive amination reaction was carried out under milder conditions than those in the study of effect of carrier (Table 2). In the case of an 80 wt% catalyst, weighing was difficult because the catalytic amount was too small, and therefore, a study was carried out with a fixed amount, specifically 20 mg. The catalytic activity greatly varied depending on the amount of cobalt supported, and in the case of a 5 wt% supported catalyst, the targeted product was not found. However, by increasing the amount supported, improvement in catalytic activity was observed, and in the case of a 20 wt% supported catalyst, the reaction proceeded almost quantitatively. When the amount supported was increased up to 80 wt%, a decrease in catalytic activity was observed.

**[Table 2]**

| Effect of amount supported | | | | | | |
|---|---|---|---|---|---|---|
| 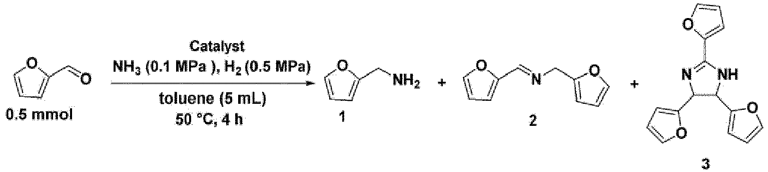 | | | | | | |
| Entry | Catalyst | Amount (mg) | Conv. (%) | Yield (%) | | |
| | | | | **1** | **2** | **3** |
| 1 | 5 wt% Co/SiO₂ | 80 | >99 | - | 85 | 6 |
| 2 | 10 wt% Co/SiO₂ | 40 | >99 | 60 | 31 | 4 |
| 3 | 20 wt% Co/SiO₂ | 20 | >99 | 96 | 1 | 1 |
| 4 | 30 wt% Co/SiO₂ | 15 | >99 | 93 | trace | 1 |
| 5 | 80 wt% Co/SiO₂ | 20 | >99 | 68 | 18 | trace |

### (3) Effect of solvent

A study of solvent was carried out with a 20 wt% supported cobalt catalyst as a catalyst (Table 3). When methanol was used, furfurylamine was obtained in better yield than toluene (entry 3). However, the color of the reaction solution was pink, and therefore, there was concern about leaching of the cobalt species. Then, the solution after the reaction was subjected to ICP-AES measurement, and as a result, it was found that the cobalt species in an amount of 1.2% based on the amount supported was eluted into the reaction solution. Moreover, a study was carried out with cyclohexane as a solvent, and as a result, difurfurylamine was produced as a by-product (entry 6). From the above results, toluene was determined as an optimum solvent taking yield and selectivity into account. In the reaction with toluene as a solvent, the reaction rate was decreased, but it can be seen that by extending the reaction time, furfurylamine was given in a high yield (entry 2).

**[Table 3]**

| Effect of solvent | | | | | |
|---|---|---|---|---|---|
| 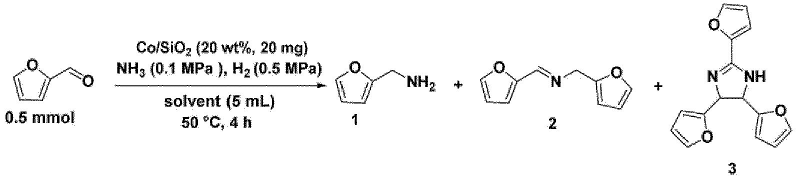 | | | | | |
| Entry | Solvent | Conv. (%) | Yield (%) | | |
| | | | **1** | **2** | **3** |
| 1 | Toluene | >99 | 53 | 37 | - |
| 2^{a} | Toluene | >99 | 97 | - | - |
| 3 | MeOH | >99 | 97 | 1 | 2 |
| 4 | Isopropanol | >99 | 26 | 47 | - |
| 5 | THF | >99 | 37 | 49 | - |
| 6 | cyclohexane | 93 | 71 | 11 | 1 |

| | | | | | |
|---|---|---|---|---|---|
| ^{a} **reaction time** 10 h | | | | | |

### (4) Study of reuse

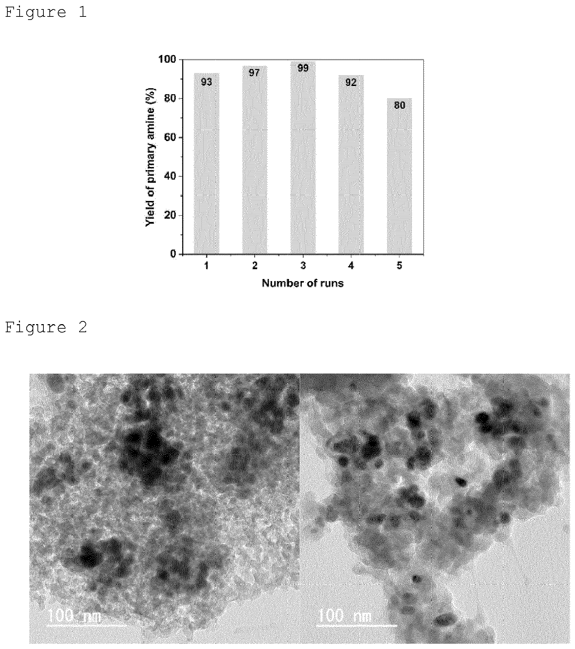

A study of reusability of a Co/SiO₂ catalyst was carried out under the optimized conditions. In the fourth reuse, slight reduction of yield was found (Figure 1). When the catalyst was observed by an electron microscope before and after the reaction, a significant difference in cobalt particle diameter between before and after the reaction was not found (Figure 2). Therefore, it is thought that this is caused by adsorption by a carbonaceous material.

### (5) Study of generality of substrate

Reductive amination reactions of various carbonyl compounds were attempted under the optimized conditions (50°C, hydrogen pressure 0.5 MPa, ammonia pressure 0.1 MPa, reaction time 15 h, toluene 5 mL, 20 wt% Co/SiO₂ 20 mg, substrate 0.5 mmol) (Table 4). It can be seen that the benzaldehyde derivative hardly received a steric effect around a formyl group. In the case of an electron donating group, the reaction efficiently proceeded, but in contrast, in the case of an electron attractive group, a reaction for a long time was needed. Even in a substrate having a bromo group or a heterocyclic aromatic ring such as pyrrole, the reaction proceeded without any problem. The present catalytic system was also applicable to aliphatic ketones or biomass-derived compounds.

**[Table 4]**

| Reductive amination reaction of carbonyl compound | | | |
|---|---|---|---|
| Entry | Substrate | Product | Yield (%) |
| 1 | 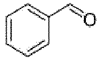 | 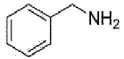 | 86 |
| 2 | 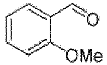 | 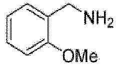 | 95 |
| 3 | 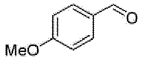 | 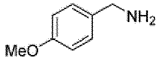 | 90 |
| 4^{a} | 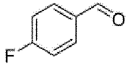 | 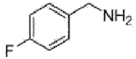 | 81 |
| 5^{a} | 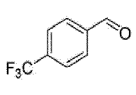 | 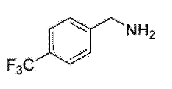 | 88 |
| 6 | 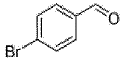 | 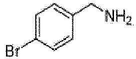 | 86 |
| 7^{a,b} | 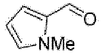 | 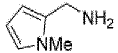 | 87 |
| 8^{c} | 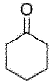 | 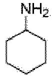 | 74 |
| 9 | 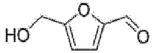 | 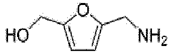 | 93 |
| ^{a} reaction time 25 hours, ^{b} hydrogen pressure 0.2 MPa, | | | |
| ^{c} using cyclohexane as reaction solvent | | | |

### (5) Comparison with precious metal catalyst

With Ru/Nb₂O₅ (Hara et al. J. Am. Chem. Soc. 2017, 139, 11493.) and Rh/Al₂O₃ (Kawanami et all. Green Chem. 2016, 18, 487.), which were precious metal catalysts as previously reported, a reductive amination reaction was attempted under the optimum conditions for the Co/SiO₂ catalyst. Regarding the amount of a metal supported, a catalyst having a supported amount that was considered optimum in literatures was prepared, and a study was carried out (Table 5). As a result, it was found that both of the catalysts exhibited a low activity as compared with the Co/SiO₂ catalyst.

**[Table 5]**

| Comparison with precious metal catalyst | | | | | |
|---|---|---|---|---|---|
| 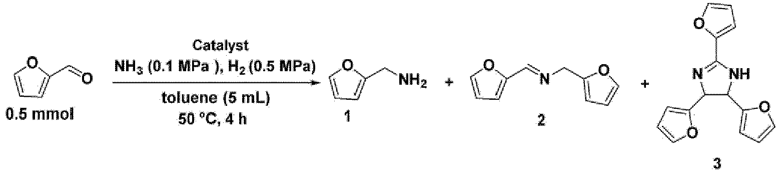 | | | | | |
| Entry | Catalyst | Conv. (%) | Yield (%) | | |
| | | | **1** | **2** | **3** |
| 1 | 20 wt% Co/SiO₂ | >99 | 53 | 37 | - |
| 2 | 1 wt% Ru/Nb₂O₅ | 93 | 36 | 38 | - |
| 3 | 5 wt% Rh/Al₂O₃ | 98 | - | 7 | 39 |

### (6) Characterization

The reduction temperature in the catalyst preparation was changed, and XRD was measured (Figure 3). It can be seen that while a state of Co₃O₄ was observed before reduction, it was reduced to CoO by reduction at 200°C. As the reduction temperature increased, a peak assigned to metallic cobalt became higher, and from 500°C, a peak assigned to CoO was hardly observed.

### (7) Concentrations of surface atoms of Co and Si

The Co/SiO₂ catalyst described in Patent Literature 1 is prepared by calcining in an air atmosphere (paragraph [0052]). On the other hand, the Co/SiO₂ catalyst of the present invention is prepared by calcining in a nitrogen stream.

It is thought that a difference in activity appears in the ratio between Co and Si in the surface. Then, the numbers of atoms of Co and Si in the surface of the Co/SiO₂ catalyst described in Patent Literature 1 (calcined in an air atmosphere) and those of the Co/Si₂ catalyst of the present invention (calcined in a nitrogen stream) were determined by X-ray photoelectron spectroscopy (XPS) (Table 6). As a result, the ratio of Co in the surface of the Co/SiO₂ catalyst of the present invention was less than 2%. On the other hand, the ratio of Co in the surface of the Co/SiO₂ catalyst described in Patent Literature 1 was about 5%. It is thought that this small number of Co atoms in the surface is one of the reasons for the high activity of the Co/SiO₂ catalyst of the present invention. The factor of the small number of Co atoms in the surface is presumed to be a structure in which the SiO₂ particles cover the Co particles, and there is a possibility that such a structure prevents cohesion or the like of the Co particles.

**[Table 6]**

| The numbers of surface atoms of Co and Si by XPS measurement | | |
|---|---|---|
| | Atomic Concentration (%) | |
| | Co | Si |
| Preparation method of the present invention | 1.92 | 98.08 |
| Preparation method of Non Patent Literature 1 | 4.93 | 95.07 |

### (8) Evaluation of electronic state of cobalt

The electronic state of cobalt supported on SiO₂ was evaluated by XPS (Co/SiO₂ in Figure 4). As a result, a peak was detected in the vicinity of 60.0 eV. On the other hand, the electronic state of cobalt given when the temperature in the hydrogen reduction was not 600°C but was 200°C was also evaluated in the same manner as above (CoO/SiO₂ in Figure 4), and as a result, a peak was detected in the vicinity of 61.6 eV. Regarding Co, a peak is detected at or in the vicinity of 60 eV, and regarding CoO, a peak is detected at or in the vicinity of 62 eV (B. Schmidt, K. Wetzig, Ion Beams in Materials Processing and Analysis (Springer, 2013)). Accordingly, it is thought that the cobalt supported on SiO₂ was not cobalt oxide but was metallic cobalt.

### [Example 2]

### (1) Relationship between amount of silanol groups of raw material SiO₂ and catalytic activity

### (1-1) Preparation of SiO₂ having different amounts of silanol groups

Into 500 mL of a 1 M nitric acid aqueous solution, 10 g of Sigma-Aldrich SiO₂ (purity > 99%) was introduced, and they were stirred at room temperature for 1 hour.

The filter residue therefrom was washed with 500 mL of distilled water three times and dried at 90°C for 12 hours. The thus prepared SiO₂ was processed under four conditions: 1) no heating, 2) heating at 400°C for 1 hour, 3) heating at 400°C for 2 hours, and 4) heating at 400°C for 5 hours, and then, the amount of silanol groups in the surface of SiO₂ was measured. Measurement of the amount of silanol groups was carried out as follows.

In a weight measurement apparatus, a sample (SiO₂) was heated at 250°C for 5 hours, and it was confirmed that the weight became constant. Thereafter, the sample was heated up to 1000°C at 1°C/min, and the weight of the sample was measured at 250°C and 1000°C. At a temperature of 600°C or more, there was no weight change of the sample. A difference between the weight of the sample at 250°C and that at 1000°C corresponded to the amount of water molecules eliminated from SiO₂ and was proportional to the amount of silanol groups in the surface of SiO₂. Accordingly, from this weight difference, the amount of silanol groups of the sample was calculated. Through the above heating, 1 mol of water molecule is eliminated from 2 mol of silanol group in the surface.

### (1-2) Preparation of catalyst and measurement of catalytic activity

By using the above-described four types of SiO₂ as carriers, catalysts were prepared in accordance with "(2) Method for preparing 20 wt% supported cobalt catalyst" of Example 1. Specifically, the method is as follows.

In 50 mL of distilled water, cobalt nitrate hexahydrate (1.23 g) and SiO₂ (1 g) were dispersed, and they were stirred at room temperature for 1 hour. Water was distilled away by a rotary evaporator, and then, the residue was dried by heating (100°C) under reduced pressure (to 0.25 kPa). The dry powder was calcined at 400°C for 2 hours in a nitrogen stream, thereby obtaining a precursor. The precursor was subjected to hydrogen reduction (30 mL/min) at 600°C for 2 hours, thereby preparing a supported cobalt catalyst. After the hydrogen reduction, the catalyst was transferred into a glove box without exposure to air, and it was used for a catalytic reaction.

With the supported cobalt catalyst prepared, a reductive amination reaction of furfural to furfurylamine was carried out. The reaction conditions were the same conditions as in "(3) Reductive amination reaction" of Example 1. That is to say, the conditions were 0.5 mmol of furfural, 0.1 MPa NH₃, 0.5 MPa H₂, 50°C, and 4 h. The isolated yield of furfurylamine is shown in the following table.

**[Table 7]**

| Heating conditions | Amount of silanol groups in surface of SiO₂ carrier as raw material (mmol/g) | Isolated yield (%) |
|---|---|---|
| Heating at 400°C for 5 hours | 3 | 53 |
| Heating at 400°C for 2 hours | 5 | 3 |
| Heating at 400°C for 1 hour | 6 | - |
| No heating | 7 | - |

As shown in the table, the catalyst prepared by using SiO₂ having 3 mmol/g of silanol groups as a raw material exhibited a high activity, but the catalysts prepared by using SiO₂ having 5 mmol/g or more of silanol groups as a raw material hardly exhibited an activity.

### (2) Relationship between surface area of raw material SiO₂ and catalytic activity

SiO₂ having been prepared by the same method as the method described in "(1) Relationship between amount of silanol groups of raw material SiO₂ and catalytic activity" of [Example 2] was subjected to heat treatment under four conditions: 1) heating at 400°C for 5 hours, 2) heating at 400°C for 10 hours, 3) heating at 500°C for 5 hours, and 4) heating at 600°C for 5 hours, and then, the surface area of SiO₂ was measured by BET method. Moreover, the amount of silanol groups in the surface of SiO₂ was also measured.

By using the above-described four types of SiO₂ as carriers, catalysts were prepared in accordance with "(2) Method for preparing 20 wt% supported cobalt catalyst" of Example 1. With the supported cobalt catalysts prepared, a reductive amination reaction of furfural to furfurylamine was carried out. The reaction conditions were the same conditions as in "(3) Reductive amination reaction" of Example 1. That is to say, the conditions were 0.5 mmol of furfural, 0.1 MPa NH₃, 0.5 MPa H₂, 50°C, and 4 h. The isolated yield of furfurylamine is shown in the following table.

**[Table 8]**

| Heating conditions | Surface area of SiO₂ carrier (m²g⁻¹) | Amount of silanol groups in surface of SiO₂ carrier as raw material (mmol/g) | Isolated yield (%) |
|---|---|---|---|
| Heating at 400°C for 5 hours | 120 | 3 | 53 |
| Heating at 400°C for 10 hours | 80 | 3 | - |
| Heating at 500°C for 5 hours | 50 | 1 | - |
| Heating at 600°C for 5 hours | <1 | 1 | - |

As shown in the table, the catalyst prepared by using SiO₂ having a surface area of 120 m²g⁻¹ as a raw material exhibited a high activity, but the catalysts prepared by using SiO₂ having a surface area of 80 m²g⁻¹ or less as a raw material hardly exhibited an activity.

### (3) Relationship among amount of silanol groups of raw material SiO₂, abundance ratio between Co⁰ and Co²⁺ in the surface, and catalytic activity

SiO₂ having silanol groups in an amount of 3 mmol/g, 5 mmol/g or 7 mmol/g and having a surface area of 120 m²g⁻¹ was prepared, and by using this as a carrier, a catalyst was prepared in accordance with "(2) Method for preparing 20 wt% supported cobalt catalyst" of Example 1. The abundance ratio between Co⁰ and Co²⁺ in the surface of cobalt of this catalyst was determined. The abundance ratio between Co⁰ and Co²⁺ in the surface was determined by measuring XPS of Co 3p, dividing peaks assigned to two of Co(0)(59 eV) and Co(2+)(62 eV), and estimating the ratio from each peak area. For the dividing, two of Gaussian function and Lorentz function were used. Moreover, with the supported cobalt catalyst prepared, a reductive amination reaction of furfural to furfurylamine was carried out. The reaction conditions were the same conditions as in "(3) Reductive amination reaction" of Example 1. That is to say, the conditions were 0.5 mmol of furfural, 0.1 MPa NH₃, 0.5 MPa H₂, 50°C, and 4 h. The isolated yield of furfurylamine is shown in the following table.

**[Table 9]**

| Surface abundance ratio between Co⁰ and Co²⁺ in surface by XPS (Co 3p) | Surface area of SiO₂ carrier (m²g⁻¹) | Amount of silanol groups in surface of SiO₂ carrier as raw material (mmol/g) | **Isolated** yield (%) |
|---|---|---|---|
| Co⁰:Co²⁺=61:39 | 120 | 3 | 53 |
| co⁰:Co²⁺=42:58 | 120 | 5 | - |
| Co⁰:Co²⁺=28:72 | 120 | 7 | - |

As shown in the table, the catalyst prepared by using SiO₂ having 3 mmol/g of silanol groups as a raw material exhibited a high activity, but the catalyst prepared by using SiO₂ having 5 mmol/g or 7 mmol/g of silanol groups as a raw material hardly exhibited an activity. As the amount of silanol groups decreased, the abundance ratio of Co⁰ became higher, and the abundance ratio of Co²⁺ became lower.

All the publications, the patents and the patent applications cited in the present specification are incorporated herein, in their entirety, by reference.

### [Industrial Applicability]

Since the catalyst of the present invention accelerates a reductive amination reaction, it can be utilized for industrial amine synthesis.

## Claims

1. A catalyst comprising cobalt supported on an oxide, the catalyst produced by a method comprising the following steps (1) to (4):
(1) a step of mixing a salt containing a cobalt ion and an oxide in water,
(2) a step of distilling water away from a mixed solution obtained in step (1) and drying a resulting solid material,
(3) a step of calcining a dried material obtained in step (2) in a nitrogen stream, and
(4) a step of reducing a calcined product obtained in step (3) in a hydrogen stream.

2. The catalyst according to claim 1, wherein an abundance ratio of metallic cobalt in a surface of the cobalt supported on the oxide is 50% or more.

3. The catalyst according to claim 1 or 2, wherein a ratio of the number of atoms of cobalt on a catalyst surface to the number of atoms of the cobalt and an oxidized element on the catalyst surface is 4% or less.

4. The catalyst according to any one of claims 1 to 3, wherein the catalyst is used in a reductive amination reaction.

5. The catalyst according to any one of claims 1 to 4, wherein the oxide is SiO₂, ZrO₂, Nb₂O₅, TiO₂, Al₂O₃, CeO₂, or MgO.

6. The catalyst according to any one of claims 1 to 4, wherein the oxide is SiO₂.

7. The catalyst according to any one of claims 1 to 4, wherein the oxide used in step (1) is SiO₂, and an amount of silanol groups in a surface of the SiO₂ is 4 mmol/g or less.

8. The catalyst according to any one of claims 1 to 4, wherein the oxide used in step (1) is SiO₂, and a surface area of the SiO₂ is 100 m²g⁻¹ or more.

9. The catalyst according to any one of claims 1 to 4, wherein the oxide used in step (1) is SiO₂, an amount of silanol groups in a surface of the SiO₂ is 4 mmol/g or less, and a surface area of the SiO₂ is 100 m²g⁻¹ or more.

10. The catalyst according to any one of claims 1 to 4, wherein the oxide used in step (1) is SiO₂, and the SiO₂ is SiO₂ having been subjected to heat treatment.

11. The catalyst according to claim 10, wherein the heat treatment is heat treatment at 350 to 480°C for 3 to 8 hours.

12. A method for producing an amine, comprising a step of obtaining, from a carbonyl compound represented by the general formula (I): 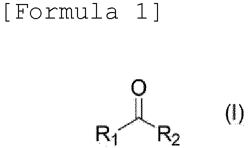 wherein R₁ and R₂ are the same or different and each represent a hydrogen atom, an aryl group optionally substituted by a substituent, or an alkyl group optionally substituted by a substituent, and R₁ and R₂ are optionally bonded to each other to form a ring,
an amine represented by the general formula (II): 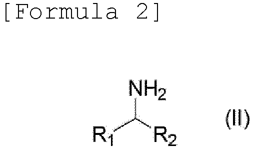 wherein R₁ and R₂ have the same meanings as above,
in the presence of the catalyst according to any one of claims 1 to 11 and a reducing agent.

13. The method for producing an amine according to claim 12, wherein the reducing agent is a hydrogen molecule.

14. The method for producing an amine according to claim 13, wherein the step of obtaining, from a carbonyl compound represented by the general formula (I), an amine represented by the general formula (II) is carried out at a temperature of 100°C or less and a hydrogen pressure of 1.0 MPa or less.

15. The method for producing an amine according to claim 14, wherein the carbonyl compound represented by the general formula (I) is furfural, and the amine represented by the general formula (II) is furfurylamine.
